# EUROPÄISCHE PATENTANMELDUNG

(11) **EP 2 184 082 A1**
(43) Veröffentlichungstag der Anmeldung: **12.05.2010**
(21) Anmeldenummer: 09172868.3
(22) Anmeldetag: 13.10.2009
(51) Int. Cl.: A61N 1/05

(54) **Implantierbare Leitung**

(30) Priorität: 06.11.2008 DE 102008043513
(71) Anmelder: Biotronik CRM Patent AG, 6341 Baar (CH)
(72) Erfinder: Dörr, Thomas, 12437, Berlin (DE)
(74) Vertreter: Lindner-Vogt, Karin L.

(57) **Zusammenfassung**

Implantierbare Leitung zur Implantation im linken Ventrikel oder linken Atrium eines Herzens unter Perforation des atrialen oder ventrikulären Septums, mit einem langgestreckten, flexiblen Leitungskörper und einer Elektrode und/oder einem Sensor und/oder einer Medikamentenabgabeeinrichtung in einem distalen, im Gebrauchszustand der Leitung im linken Atrium oder linken Ventrikel liegenden Abschnitt des Leitungskörpers, wobei der Teil des Leitungskörpers, der im Gebrauchszustand im linken Ventrikel oder linken Atriums liegt, mindestens teilweise mit einer Oberflächenbeschichtung oder Oberflächenstruktur versehen ist, die das Einwachsen mit körpereigenem Gewebe fördert.

## Beschreibung

Die Erfindung betrifft eine implantierbare Leitung, insbesondere eine Elektrodenleitung, eine Sensorleitung oder Medikamentenzufuhrleitung, die zur Implantation in einem Blutgefäß im oder am Herzen, insbesondere zur Implantation im linken Ventrikel oder linken Atrium eines Herzens unter Perforation eines entsprechenden Septums vorgesehen ist.

Als Stand der Technik für eine linksventrikuläre Stimulation und Wahrnehmung ist derzeit die Implantation einer Elektrode in eine linksventrikuläre Vene via Koronarsinus anzusehen. Diese sog. Koronarsinuselektroden kommen vorwiegend für die kardiale Resynchronisationstherapie zum Einsatz.

In der Literatur finden sich vermehrt Fallberichte von transseptaler Implantation linksventrikulären Stimulationselektroden zur Resynchronisationstherapie. Diese Implantationstechniken wurden immer unter Zuhilfenahme vorhandener Katheter, Führungsdrähte und normaler, d.h. rechtsventrikulärer Elektroden oder auch versehentlich durchgeführt. Als Zugang zum linken Ventrikel wurden entweder die Punktion des atrialen Septums oder des Ventrikelseptums beschrieben, vgl. z.B. van Gelder BM, Scheffer MG, Meijer A, et al. Transseptal endocardial left ventricular pacing: An alternative technique for coronary sinus lead placement in cardiac resynchronization therapy. [Journal Article Heart Rhythm 2007 Apr; 4(4):454-60*.*

Weitere Anwendungen von dauerimplantierten Elektroden sind die Applikation von Sensoren im linken Atrium oder Ventrikel oder aber auch eine linksseitige Medikamentenapplikation mittels dauerimplantierter Katheter.

Die Implantation einer linksventrikulären/linksatrialen Elektrode wird derzeit nicht durchgeführt, da erhebliche Bedenken hinsichtlich des Risikos von Thrombenbildungen an dieser Elektrode und dem damit verbundenen Schlaganfallrisiko bestehen. Zwar sprechen die linksventrikulären/linksatrialen Strömungsgeschwindigkeiten gegen eine Thrombenbildung, jedoch können durch die Elektrode Strömungsartefakte mit einer Blutflussgeschwindigkeit kleiner <0,4m/sec verursacht werden. Bestehen diese Verhältnisse über eine längere Zeit, dann kann an diesen Arealen eine Thrombenbildung beginnen.

Gleiches gilt für die weiter genannten Anwendungen von "drahtgebundenen" Sensoren oder Kathetern zur linksseitigen Medikamentenabgabe im Herzen.

Um diese Nachteile zu beheben, ist es Aufgabe der Erfindung, einen Katheter bzw. eine Sonde zur linksventrikulären/linksatrialen Dauerimplantation derart zu konstruieren, dass das Risiko einer Thrombenbildung im Bereich des linken Ventrikels oder Atriums deutlich reduziert und damit der Einsatz einer dauerhaft implantierten linksventrikulären bzw. linksatrialen Sonde möglich wird.

Diese Aufgabe wird durch eine Leitung mit den Merkmalen des Anspruchs 1 gelöst. Zweckmäßige Fortbildungen des Erfindungsgedankens sind Gegenstand der abhängigen Ansprüche.

Die Erfindung basiert auf einem Gedanken, dass die Gefahr einer Thrombenbildung bei einer vollständig von körpereigenem Gewebe umschlossenen Sonde wesentlich herabgesetzt ist.

Eine Leitung bzw. ein Katheter zur dauerhaften Implantation in ein Blutgefäß im oder am Herzen zur Wahrnehmung oder Abgabe elektrischer Signale oder als Sensor oder zur intrakardialen Medikamentenabgabe umfasst erfindungsgemäß einen langgstreckten, flexiblen Sondenkörper mit wenigstens einem elektrischen Pol und/oder wenigstens einem Sensor und/oder wenigstens eines Medikamentenapplikators in das Blutgefäß im oder am Herzen, und bevorzugt einer Fixierungsmöglichkeit der Sonde in einem Wandabschnitt des Blutgefäßes im oder am Herzen (Schraube, Widerhaken, ...), wobei der Teil des Elektrodenkörpers, der im Gebrauchszustand in das Blutgefäß reicht, mit einer Oberflächenbeschichtung oder Oberflächenstruktur versehen ist, die das Einwachsen des Sondenteils mit körpereigenem Gewebe fördert.

Bei einem Blutgefäß im oder am Herzen handelt es sich hierbei um Herzkranzgefäße wie beispielsweise der Koronasinusvene, Pulmonalvene oder Aorta. Mehr bevorzugt handelt es sich aber um die Herzkammern wie die rechte und linke Herzkammer oder den rechten oder linken Vorhof. Insbesondere soll der Katheter oder die Leitung dauerhaft im linken Atrium oder im linken Ventrikel implantiert sein, und zwar unter Perforation des atrialen oder ventrikulären Septums wie beispielsweise aus der DE 10 2008 040 304.0 bekannt. Möglich ist auch eine Perforation des Ventrikulären Myokards beispielsweise ausgehend von einem Herzkranzgefäß oder von außen durch das Epikard.

Bevorzugt ist die das Einwachsen fördernde Oberflächenbeschichtung oder Oberflächenstruktur in jenem Bereich des Leitungskörpers vorgesehen, der im Gebrauchszustand nahe einer Wandung des linken Ventrikels oder linken Atriums liegt.

Ein Vorteil der erfindungsgemäßen Lösung ist die Reduktion des thrombotischen Risikos einer im Blutgefäß im oder am Herz dauerhaft implantierten Sonde. Dadurch wird die Anwendung einer solchen Sonde erst ermöglicht.

Vorteile für die linksventrikuläre Stimulation sind:
- physiologisch günstigerer Stimulationsort,
- bessere Sensingsignale aufgrund der größeren Muskelmasse,
- günstigere Bedingungen zu Sondenfixierung und geringeres Perforationsrisiko aufgrund der größeren Wandstärke,
- bessere Möglichkeiten der haemodynamischen Optimierung durch Stimulation,
- Nachteile der RV-Stimulation werden weitgehend aufgehoben.

Die Erfindung ermöglicht unter Anderem die Einführung neuartiger Elektroden und damit eines neuen Stimulationskonzeptes im CRM-Bereich. Weiterhin wird die Applikation von Sensoren (z.B. Druck) oder Medikamentenkathetern zur dauerhaften linksventrikulären/linksatrialen Implantation ermöglicht.

Die Elektrode enthält in einer Ausführungsform eine "stentartige" Struktur zur Stimulation des Einschlusses mit körpereigenem Gewebe.

Die Oberflächenstruktur oder -beschichtung ist in einer Ausführungsform aus einem resorbierbaren Material hergestellt, das nach Abschluss des Einschlusses mit körpereigenem Gewebe vollständig resorbiert wird (z.B. Magnesium, Magnesiumlegierung wie WE 43 oder ein bioresorbierbares Polymer wie Poly-L-Lactid).

Im Falle eines bioresorbierbaren Polymers als Oberflächenstruktur oder -beschichtung enthält das Polymer einen Wirkstoff zur Förderung des Einwachsens oder zusätzlich oder alternativ einen Wirkstoff zur Inhibierung der Plättchenaggregation bis zum vollständigen Einschluss mit körpereigenem Gewebe.

Die Oberflächenbeschichtung besteht in einer weiteren Ausführungsform aus Siliziumkarbid (SiC).

Die Oberflächenbeschichtung oder die Oberflächenstruktur ist in einer Ausführungsform auf einem Trägermaterial, eingebettet in den flexiblen Leitungskörper, aufgebracht, welches bevorzugt eine durchgängige Schicht ist, die sich zwischen dem Leitungskörper und der Oberflächenbeschichtung oder -struktur befindet. In einer Ausführungsform enthält das Trägermaterial Wirkstoffe zur Förderung des Einwachsens in einer Depot-Einlagerungsform.

In einer weiteren Ausführungsform enthält das Trägermaterial alternativ oder zusätzlich geeignete Wirkstoffe zur Inhibierung der Plättchenaggregation bis zum vollständigen Einschluss mit körpereigenem Gewebe.

Der Sondenteil, welcher sich im Blutgefäß im oder am Herz befindet, ist in einer Ausführungsform derart vorgeformt, dass die Sonde weitgehend am Myokard anliegt (Kontakt zum Substrat zur Förderung des Einschlusses mit körpereigenem Gewebe). Sinnvollerweise wird die Elektrode im genannten Bereich derart vorgeformt (J-, U- oder S-förmig), dass diese nach der Implantation zumeist an der Wand bzw. dem Septum des linken Ventrikels anliegt. So wird die vollständige Umschließung mit körpereigenem Gewebe stimuliert. Bevorzugt ist dabei die Lage eines mit Oberflächenbeschichtung versehenen Bereiches auf die vorgeprägte Krümmung des Leitungskörpers derart abgestimmt, dass ein Abschnitt des Leitungskörpers die Oberflächenbeschichtung oder -struktur trägt, die im Gebrauchszustand Wandkontakt mit der Wand des Blutgefäßes im oder am Herz hat.

Die Oberflächenbeschichtung besteht in einer Ausführungsform aus mechanischen Mikrostrukturen mit einer Strukturgröße zwischen 1 und 500, bevorzugt zwischen 5 und 200 und noch bevorzugter zwischen 10 und 50 µm (ideal ∼16µm).

Zu einer weiteren Ausführung besteht die Oberflächenbeschichtung aus mechanischen Makrostrukturen (z.B. einer Wendelstruktur, Netzstruktur, Wabenstruktur o. ä.).
Die Leitung kann bevorzugt mehrere Abfühl- und/oder Stimulations- und/oder Schockelektroden umfassen, wobei bevorzugt mindestens ein Teil der Elektrode mit der das Einwachsen fördernden Oberflächenbeschichtung oder -struktur, insbesondere einer stent-artigen oder netzartigen oder Wendelstruktur, versehen ist.

Vorteile und Zweckmäßigkeiten in der Erfindung ergeben sich im Übrigen aus der nachfolgenden Beschreibung von Ausführungsbeispielen und -aspekten der Erfindung anhand der Figuren. Von diesen zeigen:
- Fig. 1: eine Gesamtansicht einer implantierten medizinischen Geräteordnung gemäß einer Ausführung der Erfindung,
- Fig. 2 und 2a: eine Elektrodenleitung gemäß einer Ausführungsform der Erfindung, mit Einführbesteck und
- Fig. 3A bis 3D: schematische Darstellungen zur Erläuterung eines Ausführungsaspektes der Erfindung.

Das nachfolgende Ausführungsbeispiel zeigt eine in naher Zukunft wichtige Anwendung, die Platzierung einer endokardialen Elektrode zur linksventrikulären Stimulation bei der kardialen Resynchronisationstherapie.

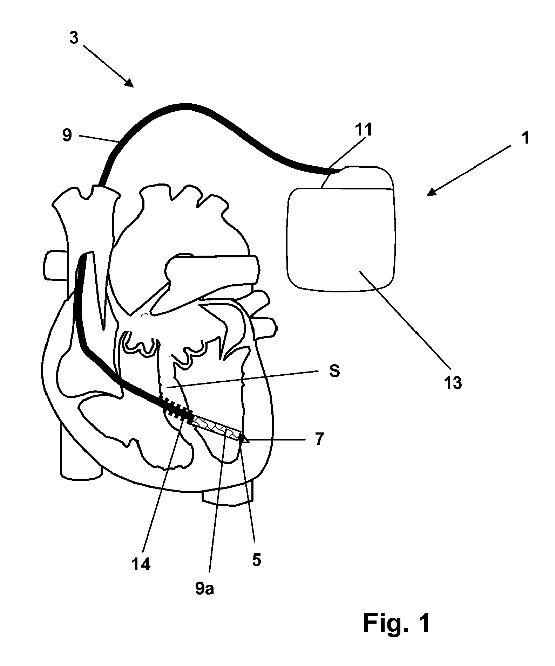

In Fig. 1 ist eine Stimulationsanordnung 1 mit einer dauerhaft linksventrikulär implantierten Stimulations- und Wahrnehmungselektrodenleitung 3 dargestellt. Die Leitung 3 besitzt einen elektrischen Pol 5 zur Stimulation des linksventrikulären Myokards, Fixierungsmittel 7 im distalen Bereich (Schraube, Tines) und einen flexiblen Leitungskörper 9, verbunden mit einem Stecker 11 zum Anschluss an ein elektronisches Implantat 13, z.B. einen Herzschrittmacher. Optional besitzt die dargestellte transseptale Elektrode einen Fixierungs-Nerschlussmechanismus 14 im Bereich des Septums S. Erfindungsgemäß ist ein Abschnitt 9a des flexiblen Leitungskörpers 9, der im linken Ventrikel liegt, mit einer SiC-Beschichtung 15 versehen, die eine Bildung körpereigenem Gewebes fördert und gleichzeitig antithrombotische Eigenschaften hat.

Eine linksatriale Anwendung ist ebenso möglich, hier wird die Elektrode durch das atriale Septum in das linke Atrium platziert.

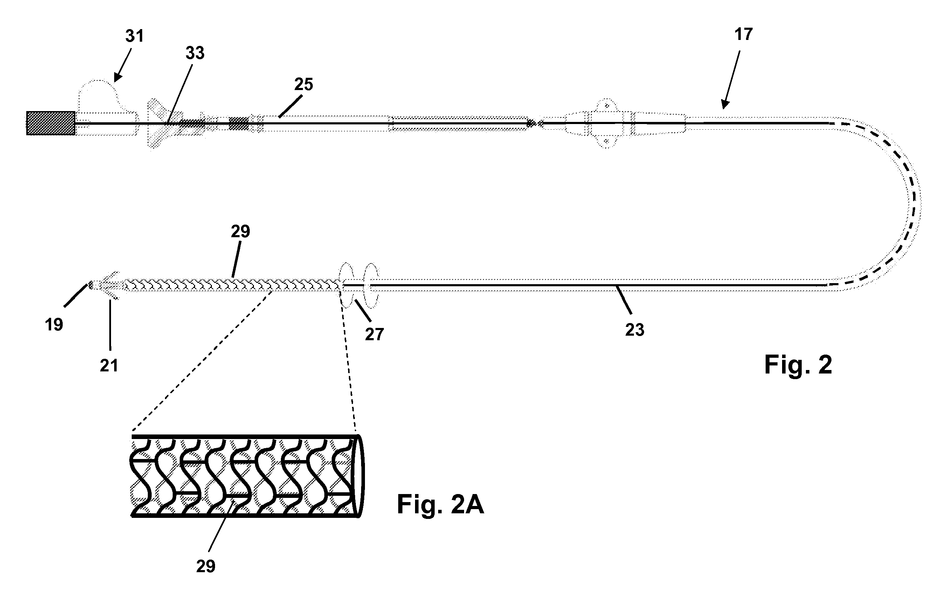

In Fig. 2 ist als Ausführungsform eine linksventrikuläre Sonde 17 dargestellt, mit einem unipolaren Stimulationspol 19, Silikon-Tines 21 zur Fixierung der Elektrode im Myokard, einem flexiblen Leitungskörper 23 und einem dem IS-1-Standard entsprechenden Anschlussstecker 25. Zusätzlich besitzt die Sonde 17 ein Verschlusselement 27, welches im implantierten Zustand im Bereich des Ventrikelseptums liegt. Um ein rasches Einwachsen der Elektrodenleitung im linken Ventrikel zu stimulieren, ist der Elektrodenkörper im Bereich des linken Ventrikels mit einem metallischen Netz 29 (vorzugsweise MP35N oder Kobalt-Chrom) überzogen bzw. ist dieses Netz in einem in den Elektrodenkörper eingebetteten Trägermaterial aufgebracht oder darin ganz oder teilweise eingebettet. Das Netz 29 ist mit Siliziumkarbit (SiC) beschichtet, welches auch amorphe Strukturen aufweisen kann (amorphes Siliziumkarbit = a:SiC).
Anstelle eines Netzes kann auch eine Wendelstruktur eingesetzt werden.

Die Figur zeigt des Weiteren ein Einführbesteck 31, welches dazu dient, die Elektrodenleitung unter Führung durch einen Führungsdraht 33 in den linken Ventrikel eines Herzens zu implantieren. Es versteht sich, dass während des Implantationsvorganges das Verschlusselement 27 durch einen geeigneten Mechanismus (der nicht zur vorliegenden Erfindung gehört) an den Leitungskörper angelegt wird, um eine widerstandsarme Hindurchführung durch die entsprechenden Gefäße und das ventrikuläre Septum zu ermöglichen.

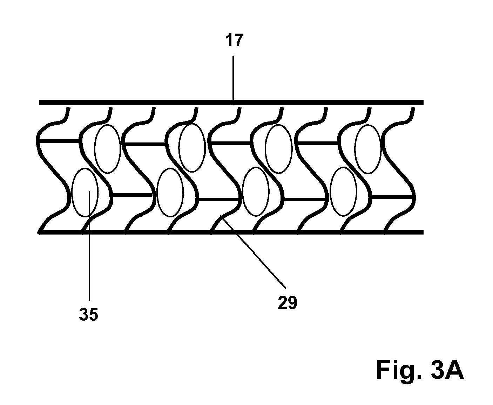

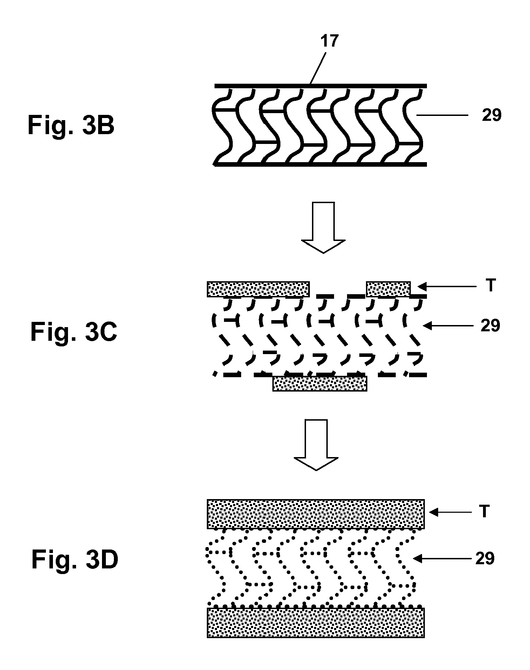

In Fig. 3A ist das im Bereich des linken Ventrikels bei der Sonde bzw. Leitung 17 aufgebrachte SiC-beschichtete Netz 29 genauer dargestellt. In dieser Applikation sind zusätzlich in den Leitungskörper 23 in diesem Bereich geeignete Wirkstoffe zur Förderung des Einwachsens und/oder Wirkstoffe zur Inhibierung der Plättchenaggregation, eingebettet in einem biodegradierbaren Trägermaterial 35, eingebracht. Die Elutionsrate ist dabei so gewählt, dass eine Freisetzung des Medikamentes für einen Zeitraum von 4-6 Wochen erfolgt. So kann bis zum Abschluss des vollständigen Umschließens der Elektrode mit körpereigenem Gewebe eine Thrombenbildung verhindert werden. Die genannten Wirkstoffe können auch in einer zwischen dem Leitungskörper und der Oberflächenbeschichtung oder -struktur befindlichen Schicht eingebracht sein. In diesem Fall sind in dieser Schicht beispielsweise Wirkstoffdepots oder mit Wirkstoff gefüllte Kavitäten eingebracht, welche eine Freisetzung der Medikamente für einen Zeitraum von 4-6 Wochen gewährleisten.

In einer weiteren Alternative kann das Netz (29) vollständig in einem biodegradierbaren Trägermaterial, welches als soeben erwähnte Schicht ausgebildet ist, komplett eingebettet sein. In diesem Fall degradiert das Trägermaterial, eluiert den oder die Wirkstoffe und setzt gleichzeitig die Oberflächenstruktur oder -beschichtung frei.

In Fig. 3B bis 3D ist skizzenartig die Wirkung eines biodegradierbaren Materials für das die Epithelialisierung fördernde Netz 29 dargestellt. Hier ist dieses Netz aus Magnesium oder einer Magnesiumlegierung (wie beim sog. AMS-Stent aus beispielsweise der Legierung WE 43) hergestellt. Mit zunehmendem Einwachsen von Körpergewebe T erfolgt die Degradierung der Magnesiumverbindung, so dass nach vollständigem Einwachsen (nach ca. 4-6 Wochen) die Netzstruktur weitgehend aufgelöst ist. Es versteht sich, dass auch bei einer solchen Oberflächenstruktur oder -beschichtung aus Magnesium oder Magnesiumlegierung geeignete Wirkstoffe zur Förderung des Einwachsens und/oder Wirkstoffe zur Inhibierung der Plättchenaggregation in einem biodegradierbaren Trägermaterial (35) oder einer zusätzlichen Schicht (wie zu Fig. 3A erläutert) eingebracht sein können. In diesem Fall kann die Schicht auch aus einer biodegradierbaren Matrix, beispielsweise aus Poly-L-Lactid hergestellt sein.

In einer weiteren Ausführung kann die Oberflächenstruktur oder -beschichtung und das Medikamentendepot kombiniert sein. In diesem Fall besteht die Oberflächenstruktur oder -beschichtung aus einem biodegradierbaren Polymer, beispielsweise Poly-L-Lacid (PLLA), in das geeignete Wirkstoffe zur Förderung des Einwachsens und/oder Wirkstoffe zur Inhibierung der Plättchenaggregation einbebettet oder eingearbeitet sind. Zusätzlich kann auch hier eine zusätzliche biodegradierbare Schicht mit denselben oder zusätzlichen Wirkstoffen vorgesehen sein.

Die Ausführung der Erfindung für linksventrikuläre/linksatriale Sensoren oder Katheter zur linksventrikulären/linksatrialen Medikamentenapplikation entspricht dem oben genannten Ausführungsbeispiel. Im Übrigen ist die Ausführung der Erfindung nicht auf die oben erläuterten Beispiele und hervorgehobenen Aspekte beschränkt, sondern ebenso in einer Vielzahl von Abwandlungen möglich, die im Rahmen fachgemäßen Handelns liegen.

## Patentansprüche

1. Implantierbare Leitung (3, 17) zur Implantation in ein Blutgefäß im oder am Herz, bevorzugt im linken Ventrikel oder linken Atrium eines Herzens unter Perforation des atrialen oder ventrikulären Septums, mit
einem langgestreckten, flexiblen Leitungskörper (9, 23) und
einer Elektrode (5, 19) und/oder einem Sensor und/oder einer Medikamentenabgabeeinrichtung in einem distalen, im Gebrauchszustand der Leitung im Blutgefäß im oder am Herzen liegenden Abschnitt des Leitungskörpers,
wobei der Teil (9a) des Leitungskörpers, der im Gebrauchszustand in dem Blutgefäß im oder am Herzen liegt, mindestens teilweise mit einer Oberflächenbeschichtung oder Oberflächenstruktur (29) versehen ist, die das Einwachsen mit körpereigenem Gewebe fördert.

2. Leitung nach Anspruch 1, wobei die das Einwachsen fördernde Oberflächenbeschichtung oder Oberflächenstruktur (29) in jenem Bereich des Leitungskörpers vorgesehen ist, der im Gebrauchszustand nahe einer Wandung des Blutgefäßes im oder am Herzen liegt.

3. Leitung nach Anspruch 1 oder 2, wobei die Oberflächenstruktur (29) oder - beschichtung ein resorbierbares Material, beispielsweise Magnesium oder Magnesiumlegierung oder ein biodegradierbares Polymer aufweist.

4. Leitung nach Anspruch 3, wobei das Polymer einen Wirkstoff zur Förderung des Einwachsens oder zusätzlich oder alternativ einen Wirkstoff zur Inhibierung der Plättchenaggregation bis zum vollständigen Einschluss mit körpereigenem Gewebe enthält.

5. Leitung nach einem Ansprüche 1 bis 3, wobei die Oberflächenstruktur (29) eine mechanische Makrostruktur, beispielsweise eine Wendelstruktur oder netzartige oder stentartige Struktur, aufweist.

6. Leitung nach einem der Ansprüche 1 bis 3, wobei die Oberflächenbeschichtung eine geometrische Mikrostruktur mit Strukturdimensionen im Bereich zwischen 1 und 500 µm, bevorzugt zwischen 5 und 200 µm und noch bevorzugter zwischen 10 und 50 µm, aufweist.

7. Leitung nach einem der vorangehenden Ansprüche, wobei die Oberflächenbeschichtung oder die Oberflächenstruktur (29) auf einem in den flexiblen Leitungskörper (23) eingebetteten Trägermaterial (35) aufgebracht ist, welches bevorzugt eine durchgehende Schicht zwischen Leitungskörper und Oberflächenbeschichtung oder Oberflächenstruktur ist.

8. Leitung nach Anspruch 7, wobei das Trägermaterial Wirkstoffe zur Förderung des Einwachsens in einer Depot-Einlagerungsform enthält.

9. Leitung nach Anspruch 7 oder 8, wobei das Trägermaterial alternativ oder zusätzlich Wirkstoffe zur Inhibierung der Plättchenaggregation bis zum vollständigen Einschluss mit körpereigenem Gewebe aufweist.

10. Leitung nach einem der vorangehenden Ansprüche, mit einem an oder nahe dem distalen Ende des Leitungskörpers (23) vorgesehenen Fixierungsmitteln (7, 21) zur Fixierung in einem Wandabschnitt in oder an dem Blutgefäß im oder am Herz.

11. Leitung nach einem der vorangehenden Ansprüche, mit mehreren Abfühl- und/oder Stimulations- und/oder Schockelektroden.

12. Leitung nach Anspruch 11, wobei mindestens ein Teil der Elektrode mit der das Einwachsen fördernden Oberflächenbeschichtung oder -struktur (29), insbesondere einer stent-artigen oder netzartigen oder Wendelstruktur, versehen ist.

13. Leitung nach einem der vorangehenden Ansprüche, wobei der Teil des Leitungskörpers (9a), der im Gebrauchszustand in oder an dem Blutgefäß im oder am Herz liegt, einen vorgeprägten Krümmungsverlauf, insbesondere in J-, S- oder U-Form, hat, welche derart ausgebildet ist, dass er durch Einführen eines Führungsdrahtes aufgehoben wird und sich nach Entfernen des Führungsdrahtes (33) und/oder durch den Einfluss von Körperwärme wieder ausbildet.

14. Leitung nach einem der vorangehenden Ansprüche, wobei die Lage eines mit Oberflächenbeschichtung oder -struktur (29) versehenen Bereiches auf die vorgeprägte Krümmung des Leitungskörpers derart abgestimmt ist, dass ein Abschnitt des Leitungskörpers (9a) die Oberflächenbeschichtung oder -struktur trägt, die im Gebrauchszustand Wandkontakt mit der Wand des Blutgefäßes im oder am Herz hat.
